# EUROPÄISCHE PATENTANMELDUNG

(11) **EP 2 112 137 A1**
(43) Veröffentlichungstag der Anmeldung: **28.10.2009**
(21) Anmeldenummer: 09157753.6
(22) Anmeldetag: 09.04.2009
(51) Int. Cl.: C07D 211/46

(54) **Verfahren zur Herstellung von quarternären Salzen von Piperidyl Estern der Mandelsäure**

(30) Priorität: 25.04.2008 DE 102008020746
(71) Anmelder: Saltigo GmbH, 40764 Langenfeld (DE)
(72) Erfinder: Job, Andreas, 50858, Köln (DE); Baskarov, Denys, 51379, Leverkusen (DE); Krahwinkel, Ralf, 40764, Langenfeld (DE); Hieronymi, Antje, 50733, Köln (DE)
(74) Vertreter: Pettrich, Klaus-Günter

(57) **Zusammenfassung**

Beschrieben wird ein Verfahren zur Herstellung von Verbindungen der allgemeinen Formel (I) 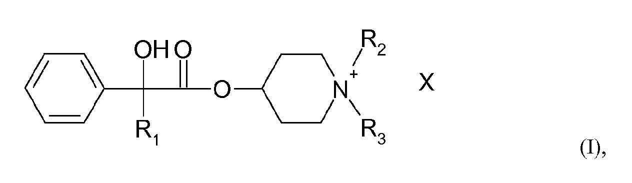 in der R₁, R₂ und R₃ unabhängig von einander für Wasserstoff, eine Alkyl- oder Arylgruppe stehen, **dadurch gekennzeichnet, dass** Verbindungen der allgemeinen Formel (II) 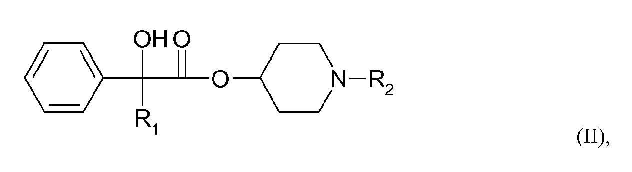 in der R₁ und R₂ die für Formel (I) angegebene Bedeutung besitzen,
mit einem Alkylierungsmittel der allgemeinen Formel (III)

R₃X (III),

in der X für ein Halogenatom steht und R₃ die für Formel (I) angegebene Bedeutung hat, gegebenenfalls in einem Lösungsmittel umgesetzt werden.

## Beschreibung

Die vorliegende Erfindung betrifft ein Verfahren zu Herstellung von substituierten quarternären Salzen von Piperidyl Estern der Mandelsäure durch die Umsetzung mit einem Alkylierungsreagenz.

Quarternäre Salze von Piperidylestern der Mandelsäure werden u.a. in pharmazeutischen Wirkstoffen, Kosmetika und Agrochemikalien eingesetzt.

Synthese von substituierten quarternären Salzen von Piperidyl Estern der Mandelsäure durch die Umsetzung von Piperidyl Estern der Mandelsäure mit einem Alkylierungsreagenz ist in der GP 788126 beschrieben. Nachteilig bei diesem Verfahren ist allerdings die Verwendung vom kanzerogenen Benzol als Lösungsmittel sowie eine niedrige Reinheit von erhaltenen Rohprodukten, die eine zusätzliche Umkristallisation von Rohprodukten erfordert. Aus der US 2,956,062 und Journal of the American Chemical Society 1956, 78, 3701 ist weiterhin bekannt, dass quarternäre Salzen von Piperidyl Estern der Mandelsäure durch die Umsetzung von Piperidyl Estern der Mandelsäure in Diethylether als Lösungsmittel mit einem Alkylierungsreagenz zugänglich sind. Im technischen Maßstab ist die Handhabung von großen Mengen Diethylether wegen der starken Tendenz zur Peroxidbildung aber mit einem hohen sicherheitstechnischen Risiko verbunden und sollte daher vermieden werden. Weiterhin ist bei diesem Verfahren auch die niedrige Reinheit von erhaltenen Rohprodukten nachteilig, die eine zusätzliche Umkristallisation von Rohprodukten erfordert.

Wegen der großen Bedeutung von substituierten quaternären Salzen von Piperidyl Estern bestand daher das Bedürfnis, ein Verfahren zu deren Herstellung bereitzustellen, das die oben geschilderten Nachteile vermeidet.

Aufgabe der vorliegenden Erfindung war daher, ein Verfahren aufzufinden, das auf möglichst einfache Weise und mit hohen Ausbeuten die Herstellung quaternärer Salze von Piperidylestern ermöglicht und das auch großtechnisch durchführbar ist.

Gegenstand der vorliegenden Erfindung ist ein Verfahren zur Herstellung von Verbindungen der allgemeinen Formel (I) 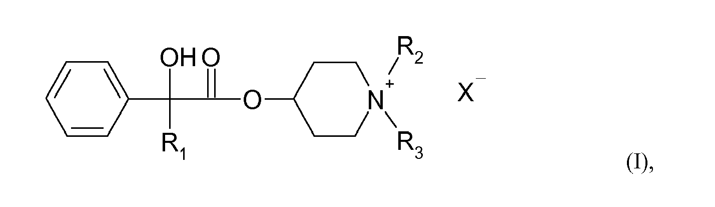 in der R₁, R₂ und R₃ unabhängig von einander für Wasserstoff, eine Alkyl- oder Arylgruppe stehen, **dadurch gekennzeichnet, dass** Verbindungen der allgemeinen Formel (II) 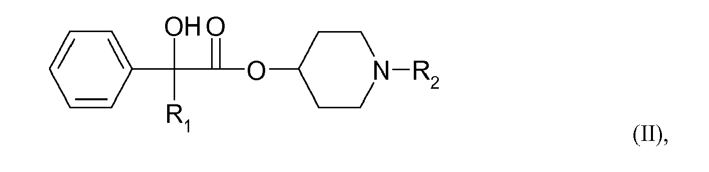 in der R₁ und R₂ vorstehend genannten Bedeutung besitzen,
mit einem Alkylierungsmittel der allgemeinen Formel (III)

R₃X (III),

in der X für ein Halogenatom steht, umgesetzt werden.

Bevorzugt steht R₁ für einen C₃C₂₀-Cycloalkylrest und R₂ für einen C₁-C₂₀-Alkylrest. Ganz besonders bevorzugt steht R₁ für ein Cyclopentyl- oder Cyclohexylrest und R₂ für einen Methyl- oder Ethylrest.

Alkylierungsmittel der Formel (II) sind Alkylhalogenide, wobei die Bromide und Iodide bevorzugt, besonders bevorzugt die Bromide sind. Bevorzugte Alkylreste R₃ sind C₁-C₂₀-Alkyl, C₃-C₂₀-Cycloalkyl. Besonders bevorzugt sind lineare C₁-C₂₀-Alkylreste. Besonders bevorzugt ist R₃ ein Methylrest, d.h. R₃-X ist in diesem Fall Methylbromid.

Die Reaktionstemperatur für das erfindungsgemäße Verfahren kann beispielsweise im Bereich von -100 bis 300°C, vorzugsweise im Bereich von -20 bis 100°C liegen. Besonders bevorzugt wird die Reaktion bei Raumtemperatur (20 bis 25°C) durchgeführt.

Der Reaktionsdruck für das erfindungsgemäße Verfahren kann beispielsweise im Bereich von 1 hPa bis 20 MPa, vorzugsweise 100 hPa bis 2 MPa liegen. Besonders bevorzugt wird die Reaktion bei Normaldruck durchgeführt.

Das erfindungsgemäße Verfahren kann in einem Lösungsmittel durchgeführt werden. Als Lösungsmittel kommen beispielsweise aprotische Lösungsmittel in Betracht, wie z.B. die Ester von Carbonsäuren wie z.B. Essigester, Butylacetat, Benzylbenzoat oder aromatische oder aliphatische halogenierte Kohlenwasserstoffe wie z.B. Chlorbenzol, Dichlorbenzol, Dichlormethan, Chloroform oder Tetrachlorkohlenstoff. Auch Nitrile, wie z.B. Acetonitril, Propionitril oder Benzonitril, oder Amide, wie z.B. N,N-Dimethylformamid, N,N-Dimethylacetamid, N-Methylformanilid, N-Methylpyrrolidon oder Hexamethylphosphorsäureamid, sowie Schwefelverbindungen wie z.B. Sulfolan und aliphatische halogenierte Kohlenwasserstoffe oder deren Mischungen. Besonders bevorzugt sind aliphatische halogenierte Kohlenwasserstoffe. Ganz besonders bevorzugt wird Dichlormethan als Lösungsmittel eingesetzt.

Es ist auch möglich, die Reaktion ohne Zusatz von Lösungsmittel durchzuführen. Das gilt insbesondere dann, wenn das Edukt der Formel (II) unter Reaktionsbedingungen flüssig ist.

Bevorzugte Eduktkonzentrationen in der Reaktionslösung liegen zwischen 1 Gew.-% und 100 Gew.-%, besonders bevorzugt zwischen 10 - 50 Gew.-%, ganz besonders bevorzugt zwischen 10 - 25 Gew.-%.

Zur Durchführung der Reaktion kann man beispielsweise so vorgehen, dass die Verbindungen der Formel (II), gegebenenfalls unter Zusatz von Lösungsmittel, vorgelegt werden und der Alkylierungsmittel der Formel (III) oder gegebenenfalls seiner Lösung in einem Lösungsmittel zugegeben wird. Alternativ ist auch die Vorlage des Alkylierungsmittels, gegebenenfalls in einem Lösungsmittel, und die anschließende Zugabe von Edukt oder seiner Lösung in einem Lösungsmittel möglich. Ebenso ist die Simultandosierung von Edukten mit der Formel (II) und des Alkylierungsmittels der Formel (III) möglich.

Auf erfindungsgemäße Weise werden in einem einzigen Schritt in hoher chemischer Ausbeute nach einer einfachen Aufarbeitung die gewünschten quarternären Salze von Piperidyl Estern der Mandelsäure erhalten.

Die erfindungsgemäß herstellbaren Verbindungen der Formel (I) eignen sich insbesondere zur Herstellung von Agrochemikalien, Pharmazeutischen Wirkstoffen, Rich- und Aromastoffen, Geschmacksstoffen, Wirkstoffen im kosmetischen Bereich, und in den Polymeren.

### Beispiele

### Beispiel 1

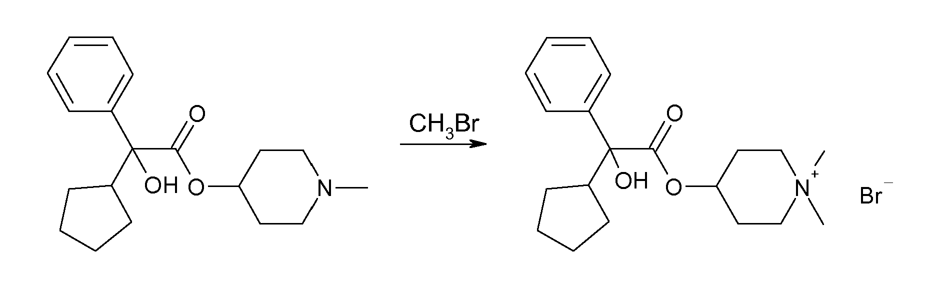

In einem 6-Liter Doppelmantelreaktor wurde Dichlormethan (2500 ml) vorgelegt und bei 5-10°C Brommethan (150 g, 1,579 mol) eingeleitet. Anschließend wurde bei 10-15°C die Lösung von α-Cyclopentyl--1-methyl-4-piperidyl Mandelsäure Ester in Heptan (100 g, 0,237 mol, 75%) zudosiert. Restliches Brommethan (401 g, 4,22 mol) wurde in das Reaktionsgemisch gleichzeitig mit der Lösung von α-Cyclopentyl--1-methyl-4-piperidyl Mandelsäure Ester (737 g, 1,744 mol, 75%) zudosiert. Die Reaktionslösung wurde auf die Raumtemperatur gebracht und über Nacht unter Stickstoff gerührt. Die Reaktionslösung wurde mit Dichlormethan (1033 g) versetzt und anschließend wurde das Lösungsmittel unter Normaldruck abdestilliert. Die Produktsuspension wurde über eine Drucknutsche filtriert und mit Dichlormethan (1550 g), Aceton (2195 g) und Ethylacetat (2195 g) gewaschen und getrocknet. Die Ausbeute betrug 804 g (98%d.Th.).

### Beispiel 2

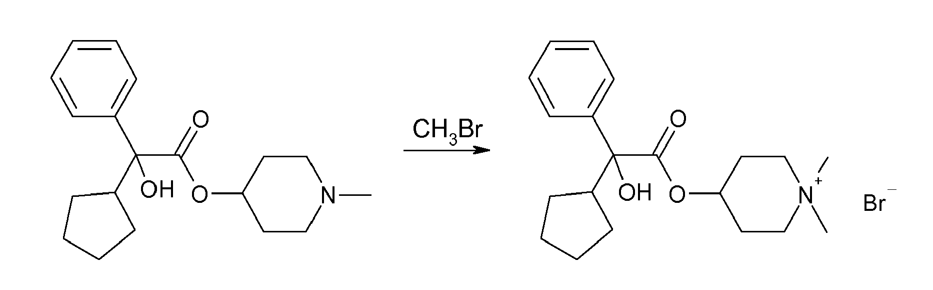

In einem 6-Liter Doppelmantelreaktor wurde die Lösung von α-Cyclopentyl-1-methyl-4-piperidyl Mandelsäure Ester (663 g, 1.61 mol, 77%) in Heptan vorgelegt und mit Dichlormethan (2500 ml) versetzt. Anschließend wurde bei 20 °C Brommethan (458 g, 4.82 mol) eingeleitet. Die Reaktionslösung wurde bei Raumtemperatur über Nacht unter Stickstoff gerührt. Das Lösungsmittel wurde zum Teil unter Normaldruck abdestilliert (943 g). Die Produktsuspension wurde über einer Drucknutsche filtriert und mit Dichlormethan (1944 g), Aceton (1590 g) und Ethylacetat (2211 g) gewaschen und getrocknet. Die Ausbeute betrug 654 g (98%d.Th.).

## Patentansprüche

1. Verfahren zur Herstellung von Verbindungen der allgemeinen Formel (I) 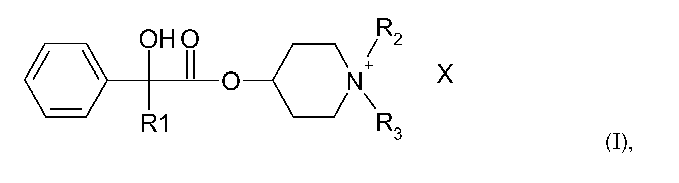 in der R₁, R₂ und R₃ unabhängig von einander für Wasserstoff, eine Alkyl- oder Arylgruppe stehen, **dadurch gekennzeichnet, dass** Verbindungen der allgemeinen Formel (II) 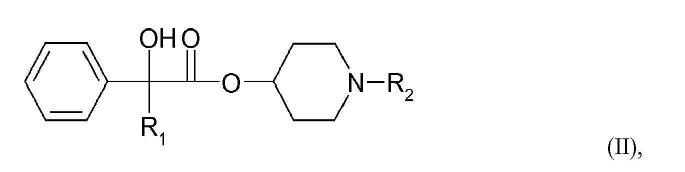 in der R₁ und R₂ die für Formel (I) angegebene Bedeutung besitzen,
mit einem Alkylierungsmittel der allgemeinen Formel (III)
R₃X (III),
in der X für ein Halogenatom steht und R₃ die für Formel (I) angegebene Bedeutung hat, gegebenenfalls in einem Lösungsmittel umgesetzt werden.

2. Verfahren nach Anspruch 1, **dadurch gekennzeichnet, dass** R₁ für einen C₃-C₂₀-Cycloalkylrest und R₂ für einen C₁-C₂₀-Alkylrest steht.

3. Verfahren nach Anspruch 1 oder 2, **dadurch gekennzeichnet, dass** R₁ für einen Cyclopentyl- und R₂ für einen Methylrest steht.

4. Verfahren nach einem der Ansprüche 1 bis 3, **dadurch gekennzeichnet, dass** X für ein Brom- oder Iod-Atom und R₃ für einen C₁-C₂₀-Alkyl oder C₃-C₂₀-Cycloalkylrest steht.

5. Verfahren nach einem der Ansprüche 1 bis 4, **dadurch gekennzeichnet, dass** X für ein Bromatom und R₃ für einen Methylrest steht.

6. Verfahren nach einem der Ansprüch 1 bis 5, **dadurch gekennzeichnet, dass** die Umstzung in Dichlormethan als Lösungsmittel erfolgt.

7. Verfahren nach einem der Ansprüche 1 bis 6, **dadurch gekennzeichnet, dass** das Alkylierungsmittel in einem Lösungsmittel vorgelegt wird und die Verbindung der Formel (II) gegebenenfalls als Lösung in einem Lösungsmittel, zudosiert wird.

8. Verfahren nach einem der Ansprüche 1 bis 7, **dadurch gekennzeichnet, dass** die Verbindung der allgemeinen Formel (II) α-Cyclopentyl-1-methyl-4-piperidyl-Mandelsäure-Ester ist.
